# EUROPEAN PATENT APPLICATION

(11) **EP 4 756 500 A1**
(43) Date of publication of application: **10.06.2026**
(21) Application number: 24852202.1
(22) Date of filing: 01.08.2024
(51) Int. Cl.: G01T 1/29, G01T 1/26, G01T 1/166, A61B 6/03

(54) **RESISTANCE PLATE DETECTOR FOR READING POSITIVE AND NEGATIVE SIGNALS WITH DIFFERENTIAL AMPLIFIERS, RESPECTIVELY**

(30) Priority: 04.08.2023 KR 20230102167
(71) Applicant: Seoul National University Hospital, Seoul 03080 (KR)
(72) Inventor: KIM, Do-Won, Seoul 06597 (KR); PARK, Sangwoo, Seongnam-si, Gyeonggi-do 13620 (KR)
(74) Representative: V.O.
(86) International application number: PCT/KR2024/011336
(87) International publication number: WO 2025/033848

(57) **Abstract**

This resistance plate detector for reading positive and negative signals with differential amplifiers, respectively, includes: a plurality of first positive (+) signal strips formed in a strip shape extending in a first direction on a first surface of a first substrate and arranged in parallel to each other; a plurality of first negative (-) signal strips formed in the same shape as the first positive signal strips on a second surface opposite to the first surface of the first substrate, facing each corresponding first positive signal strip in pairs, and arranged in parallel to each other in the first direction; and a plurality of first ground strips formed in the same shape between the plurality of first positive signal strips and the plurality of first negative signal strips and arranged in parallel to each other in the first direction, wherein output signals of a pair of a first positive signal strip and a first negative signal strip, which correspond to each other, are input to one differential amplifier. In the present invention, the ground strips have a rectangular shape or a modified shape thereof, like the signal strips, thus reducing noise of the resistance plate detector to thereby improve performance.

## Description

### TECHNICAL FIELD

The present disclosure relates to a resistance plate detector for reading signals using a strip pair, and more particularly, to technology for reducing load and noise in a resistance plate detector applied to Time-of-flight (TOF) Positron Emission Tomography (PET).

### [Cross-Reference to Related Applications]

This application is based on and claims priority from Korean Patent Application No. 10-2023-0102167 filed on August 4, 2023 with the Korean Intellectual Property Office, the disclosure of which is incorporated herein by reference in its entirety.

### BACKGROUND ART

Positron Emission Tomography (hereinafter PET) tracks the location of positron emissions from outside the body by using detectors after injection of radiopharmaceuticals containing positron-emitting radioactive isotopes into the body.

Through tracking, the patient's condition may be identified in a noninvasive manner by analyzing the activity of the injected radiopharmaceuticals in the body. Accordingly, PET technology is widely used in a variety of medical tests to identify patients' conditions such as cancers, heart diseases, brain diseases or brain functions.

PET observes two gamma rays emitted in approximately 180° directions after annihilation by collision of positrons emitted from the injected radioactive substances and the surrounding electrons, obtains data corresponding to the line integrals of the positron distribution and calculates positron distribution images using the data.

Specifically, when two PET detectors detect gamma rays from the same positron-electron collision at t1 and t2, it signifies that one positron exists on the line-of-response (LOR) connecting the corresponding two detectors, and the line integral value on the corresponding LOR of the positron distribution may be obtained.

Filtered Backprojection (FBP) or Expectation-Maximization (EM) is widely used to calculate the distribution images from the line integral values.

From the early stage of PET development, many studies have been conducted to improve image reconstruction by more accurate estimation of the positron location on the corresponding LOR using the time-of-flight (TOF) t = t1 - t2 information observed from the detectors, but it was challenging due to the low time resolution of PET.

PET-related technology improvements from the 2000s made it possible to be applied to image reconstruction using time resolution. Currently, pixels are used in a manner of obtaining signals in resistance plate detectors having high time resolution.

However, when pursuing the desired position resolution on a plane in the resistance plate detectors, the pixel-based method requires a very large number of channels (n ∝ x × y), resulting in high load and noise.

### DISCLOSURE

### Technical Problem

The present disclosure is designed to address the above-described problems, and therefore the present disclosure is directed to providing a resistance plate detector for reading each of positive and negative signals with a differential amplifier to reduce load and noise.

### Technical Solution

To achieve the above-described objective of the present disclosure, a resistance plate detector for reading each of positive and negative signals with a differential amplifier according to an embodiment includes a plurality of first positive (+) signal strips formed in a shape of a strip that extends in a first direction on a first surface of a first substrate, and arranged parallel to each other; a plurality of first negative (-) signal strips formed in a same shape as the first positive signal strips on a second surface opposite the first surface of the first substrate, and arranged parallel to each other in the first direction such that each first negative signal strip and each corresponding first positive signal strip face each other in pair; and a plurality of first ground strips formed in a same shape between the plurality of first positive signal strips and the plurality of first negative signal strips, and arranged parallel to each other in the first direction, wherein output signals of the pair of the first positive signal strip and the corresponding first negative signal strip are input to one differential amplifier.

In an embodiment of the present disclosure, each of the plurality of first positive signal strips, the plurality of first negative signal strips and the plurality of first ground strips may be formed with a same shape, width, length, thickness and interval.

In an embodiment of the present disclosure, the plurality of first positive signal strips, the plurality of first negative signal strips and the plurality of first ground strips may be formed with a same shape, width, length, thickness and interval.

In an embodiment of the present disclosure, each of the first positive signal strip and the first negative signal strip may have two ends connected to at least one resistor and at least one capacitor.

In an embodiment of the present disclosure, a width and interval of the plurality of first positive signal strips and the plurality of first negative signal strips may be adjusted according to a target input impedance of the differential amplifier.

In an embodiment of the present disclosure, the resistance plate detector for reading each of positive and negative signals with the differential amplifier may further include a plurality of second positive signal strips formed in a shape of a strip on a first surface of a second substrate and arranged parallel to each other, the first surface of the second substrate spaced a predetermined distance apart from the first surface of the first substrate, facing the first surface of the first substrate; a plurality of second negative signal strips formed in a same shape as the second positive signal strips on a second surface opposite the first surface of the second substrate, and arranged parallel to each other such that each second negative signal strip and each corresponding second positive signal strip face each other in pair; and a plurality of second ground strips formed in a same shape between the plurality of second positive signal strips and the plurality of second negative signal strips, and arranged parallel to each other.

In an embodiment of the present disclosure, the plurality of second positive signal strips, the plurality of second negative signal strips and the plurality of second ground strips may extend in a second direction perpendicular to the first direction.

In an embodiment of the present disclosure, output signals of the pair of the second positive signal strip and the corresponding second negative signal strip may be input to one differential amplifier, and a width and interval of the plurality of second positive signal strips and the plurality of second negative signal strips may be adjusted according to a target input impedance of the differential amplifier.

In an embodiment of the present disclosure, each of the plurality of first positive signal strips, the plurality of first negative signal strips, the plurality of second positive signal strips, the plurality of second negative signal strips, the plurality of first ground strips and the plurality of second ground strips may have at least one of a rectangular shape, an extended rectangular shape that narrows into a triangular shape at two ends, or an extended rectangular shape that narrows slantly at one end.

In an embodiment of the present disclosure, the resistance plate detector for reading each of positive and negative signals with the differential amplifier may be applied to Time-of-flight (TOF) Positron Emission Tomography (PET).

### Advantageous Effects

According to the resistance plate detector for reading each of positive and negative signals with the differential amplifier, it may be possible to reduce load and noise in signals, thereby improving the performance of Time-of- flight (TOF) Positron Emission Tomography (PET) applied.

### BRIEF DESCRIPTION OF THE DRAWINGS

FIG. 1 is a schematic perspective view illustrating a resistance plate detector for reading each of positive and negative signals with a differential amplifier according to an embodiment of the present disclosure.
FIG. 2 is a detailed perspective view of the resistance plate detector of FIG. 1.
FIG. 3 is an exemplary cross-sectional circuit diagram of the resistance plate detector of FIG. 2.
FIG. 4 is an exemplary plan circuit diagram of the resistance plate detector of FIG. 2.
FIGS. 5 to 7 show the shapes of strips of FIG. 2.
FIG. 8 is a schematic plan view of the resistance plate detector of FIG. 2.

### DESCRIPTION OF EMBODIMENTS

The present disclosure will be described in detail below with reference to the accompanying drawings showing particular embodiments for illustrative purposes. These embodiments are described in sufficient detail to enable those skilled in the art to practice the invention. It should be understood that various embodiments of the present disclosure are different but not mutually exclusive. For example, specific shapes, structures and features as described herein may be realized in other embodiments without departing from the scope of the present disclosure. Furthermore, it should be understood that the location or position of individual components in each of the disclosed embodiments may be changed without departing from the scope of the present disclosure. Accordingly, the following detailed description is not intended to be limiting, and when appropriately described, the scope of the present disclosure is defined by the full scope of the appended claims and their equivalents. In the drawings, like reference numerals indicate the same or similar functions across different aspects.

When an element or layer is referred to as being "on" another element or layer, it can be directly on the other element or layer and intervening layers or elements may be present. In contrast, when an element is referred to as being "directly on" another element, intervening elements are absent.

The spatially relative term such as "below", "beneath", "lower", "above" or "upper" may be used to easily describe the relationship between one element or component and other elements or components as shown in the drawings. The spatially relative terms should be understood as the terms including not only the directions shown in the drawings but also different directions of the elements during use or operation. For example, when inverted, an underlying element may be placed "above". Accordingly, the term "below" or "beneath" may include "above" as well. The elements may be oriented in other directions, and accordingly the spatially relative terms may be interpreted according to the orientation.

Although the terms first, second or the like are used to describe various elements, components and/or sections, these elements, components and/or sections are not limited by these terms. These terms are used to distinguish one element, component or section from other elements, components or sections. Accordingly, it should be understood that a first element, a first component or a first section as used herein may be a second element, a second component or a second section within the technical aspects of the present disclosure.

The terminology as used herein is provided to describe the embodiments but not intended to limit the present disclosure. In this specification, the singular form includes the plural forms unless the context clearly dictates otherwise. The term "comprises" and/or "comprising" as used herein specifies the stated components, steps, operations and/or elements, and does not exclude the presence or addition of one or more other components, steps, operations and/or elements.

Unless otherwise defined, all terms (including technical and scientific terms) as used herein may be used in the common sense to persons having ordinary skill in the technical field pertaining to the present disclosure. Furthermore, the terms defined in commonly used dictionaries should not be interpreted in an ideal or overly way unless explicitly and specifically defined otherwise.

Hereinafter, embodiments of the present disclosure will be described in detail with reference to the accompanying drawings, and in describing with reference to the accompanying drawings, the same reference numerals are affixed to the same or corresponding components regardless of the reference signs, and an unnecessary repetition of description is omitted to avoid redundancy.

Hereinafter, exemplary embodiments of the present disclosure will be described in more detail with reference to the accompanying drawings.

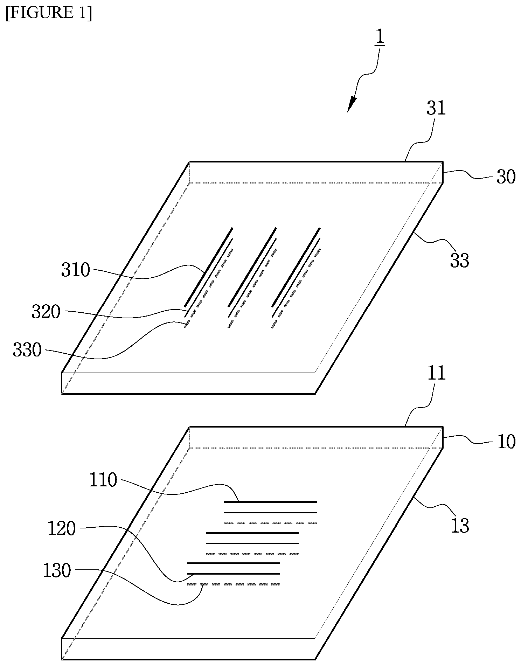

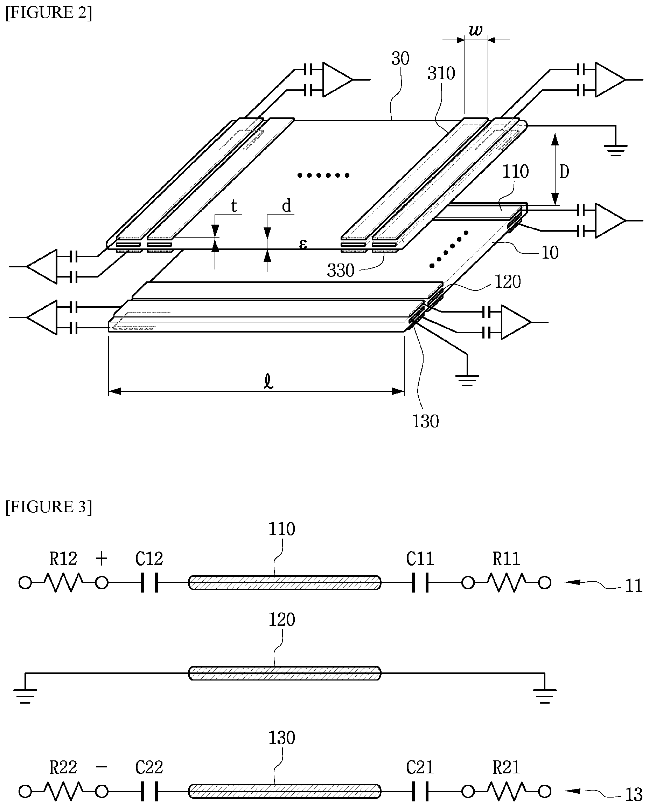

FIG. 1 is a schematic perspective view illustrating a resistance plate detector for reading each of positive and negative signals with a differential amplifier according to an embodiment of the present disclosure. FIG. 2 is a detailed perspective view of the resistance plate detector of FIG. 1.

The resistance plate detector 1 according to the present disclosure is a device that outputs a signal for finding the location of positrons when it is applied to Time-of-flight Positron Emission Tomography (TOF-PET). The resistance plate detector 1 may be a device that is separated from TOF-PET, or may be a module of the device.

Referring to FIGS. 1 and 2, the resistance plate detector 1 according to the present disclosure includes a plurality of positive (+) signal strips 110 and a plurality of negative (-) signal strips 130 that form pairs on each of two surfaces of a first substrate 10, and a plurality of corresponding ground strips 120 between the corresponding positive signal strips 110 and negative signal strips 130.

The plurality of first positive signal strips 110, the plurality of first negative signal strips 130 and the plurality of first ground strips 120 extend in a direction (a first direction) and are arranged parallel to each other.

Each of output signals of the pair of the first positive signal strip 110 and the first negative signal strip 130 is used as input of one differential amplifier. The width and interval of the plurality of first positive signal strips 110 and the plurality of first negative signal strips 130 may be adjusted according to the target input impedance of the differential amplifier.

In the present disclosure, because the plurality of first ground strips 120 are in the shape of a strip, not a single plane, the signals do not spread out from the ground and escape in a straight line shape along the ground strips, thereby reducing interference between channels and reducing signal noise.

For the sake of convenience, the first substrate 10 is referred to as an x-plane, and a second substrate 30 is referred to as a y-plane. The first substrate 10 has a first surface 11 and an opposite, second surface 13, and the second substrate 30 has a first surface 33 and an opposite, second surface 31.

For example, the first substrate 10 and the second substrate 30 may be a Printed Circuit Board (PCB) substrate with a predetermined thickness (d) having a dielectric constant ε. The first substrate 10 and the second substrate 30 may be single-layered or multi-layered. In addition, the first substrate 10 and the second substrate 30 may be parallel to each other with a predetermined distance D between them.

In the present disclosure, an upper surface of the first substrate 10 is defined as the first surface 11, and a lower surface is defined as the second surface 13. In addition, a lower surface of the second substrate 30 facing the first surface 11 of the first substrate 10 is defined as the first surface 33, and an upper surface of the second substrate 30 is defined as the second surface 31. However, these definitions are provided for convenience of description, and in the substrate or surface, upper, lower, left and right may be subject to change according to location, selection or need.

The plurality of first positive signal strips 110 are formed in the shape of a strip that extends in the x-axis direction on the first surface 11 of the first substrate 10, and arranged parallel to each other.

The plurality of first positive signal strips 110 may be formed in the shape of an extended rectangle or its variation, and may be formed with the same shape, width (w), length (1) and thickness (t). In addition, the plurality of first positive signal strips 110 may be formed at an equal interval on the first surface 11 of the first substrate 10.

For example, the width (w) of the first positive signal strip 110 may be 4 mm, the length (l) of the first signal strip 110 may be 160 mm, the thickness (t) of the first signal strip 110 may be 0.5 Oz, and the interval between the first signal strips 110 may be 1 mm. However, these dimensions may be changed according to the design.

The plurality of first negative signal strips 130 are formed on the second surface 13 of the first substrate 10 in the same shape as the plurality of first positive signal strips 110, and are arranged such that each first negative signal strip 130 and its corresponding first positive signal strip face each other in pair.

In other words, the first positive signal strips 110 and the first negative signal strips 130 formed in the first substrate 10 may be formed at overlapping locations on opposite surfaces and matched to each other in a one-to-one correspondence relationship. For example, the first positive signal strips 110 and the corresponding first negative signal strips 130 may form a total of 32 pairs, forming 32 channels.

Likewise, the plurality of first negative signal strips 130 are formed in the shape of a strip that extends in the x-axis direction on the second surface 13 of the first substrate 10, and arranged parallel to each other.

The plurality of first negative signal strips 130 may be formed in the shape of an extended rectangle or its variation, and may be formed with the same shape, width (w), length (l) and thickness (t). In addition, the plurality of first negative signal strips 130 may be formed at an equal interval on the second surface 13 of the first substrate 10.

The plurality of first ground strips 120 are formed in the shape of a strip that extends in the x-axis direction within the first substrate 10, and arranged parallel to each other.

The plurality of first ground strips 120 may be formed in the shape of an extended rectangle or its variation, and may be formed with the same shape, width (w), length (l) and thickness (t). In addition, the plurality of first ground strips 120 may be formed at an equal interval.

In an embodiment, the first positive signal strips 110, the first negative signal strips 130 and the first ground strips 120 formed in the first substrate 10 may be formed with the same shape and size in the x-axis direction so as to overlap each other.

Although this embodiment describes the plurality of first positive signal strips 110 formed on the first surface 11 of the first substrate 10 and the plurality of first negative signal strips 130 formed on the second surface 13 of the first substrate 10 for the sake of convenience, the plurality of first positive signal strips 110 may be formed on the second surface 13 and the plurality of first negative signal strips 130 may be formed on the first surface 11.

As an embodiment, the resistance plate detector 1 may further include the second substrate 30 including a plurality of second positive signal strips 330 and a plurality of second negative signal strips 310 on opposite surfaces, respectively.

Likewise, second ground strips 320 may be formed between the corresponding second positive signal strips 330 and second negative signal strips 310.

The plurality of second positive signal strips 330, the plurality of second negative signal strips 310 and the plurality of second ground strips 320 extend in a direction (a second direction) and are arranged parallel to each other.

In an embodiment, the plurality of second positive signal strips 330, the plurality of second negative signal strips 310 and the plurality of second ground strips 320 formed in the second substrate 30 may be formed in the second direction perpendicular to the first direction.

Each of output signals of the pair of the second positive signal strip 330 and the second negative signal strip 310 is used as input of one differential amplifier. The width and interval of the plurality of second positive signal strips 330 and the plurality of second negative signal strips 310 may be adjusted according to the target input impedance of the differential amplifier.

In the similar way to the plurality of first positive signal strips 110, the plurality of second positive signal strips 330 may be formed in the shape of an extended rectangle or its variation, and may be formed with the same shape, width (w), length (l) and thickness (t). In addition, the plurality of second positive signal strips 330 may be formed at an equal interval on the first surface 33 of the second substrate 30.

In an embodiment, the plurality of second positive signal strips 330 may be formed with the same shape, width (w), length (l) and thickness (t) as the plurality of first positive signal strips 110.

The plurality of second negative signal strips 310 are formed on the second surface 31 of the second substrate 30 in the same shape as the plurality of second positive signal strips 330, and are arranged such that each second negative signal strip 310 and its corresponding second positive signal strip 330 face each other in pair.

In other words, the second positive signal strips 330 and the second negative signal strips 310 formed in the second substrate 30 may be formed at overlapping locations on opposite surfaces and matched to each other in a one-to-one correspondence relationship. For example, the second positive signal strips 330 and the corresponding second negative signal strips 310 may form a total of 32 pairs, forming 32 channels.

Likewise, the plurality of second negative signal strips 310 may be formed in the shape of a strip that extends in the y-axis direction on the second surface 31 of the second substrate 30, and arranged parallel to each other.

The plurality of second negative signal strips 310 may be formed in the shape of an extended rectangle or its variation, and may be formed with the same shape, width (w), length (1) and thickness (t). In addition, the plurality of second negative signal strips 310 may be formed at an equal interval on the second surface 31 of the second substrate 30.

In an embodiment, the plurality of second negative signal strips 310 may be formed with the same shape, width (w), length (1) and thickness (t) as the plurality of first negative signal strips 130.

The plurality of second ground strips 320 are formed in the shape of a strip that extends in the y-axis direction within the second substrate 30, and arranged parallel to each other.

The plurality of second ground strips 320 may be formed in the shape of an extended rectangle or its variation, and may be formed with the same shape, width (w), length (1) and thickness (t). In addition, the plurality of second ground strips 320 may be formed at an equal interval.

In an embodiment, the plurality of second ground strips 320 may be formed with the same shape, width (w), length (1) and thickness (t) as the plurality of first ground strips 120.

In an embodiment, the second positive signal strips 330, the second negative signal strips 310 and the second ground strips 320 formed in the second substrate 30 may be formed with the same shape and size in the y-axis direction so as to overlap each other.

Although this embodiment describes the plurality of second positive signal strips 330 formed on the first surface 33 of the second substrate 30 and the plurality of second negative signal strips 310 formed on the second surface 31 of the second substrate 30 for the sake of convenience, the plurality of second positive signal strips 330 may be formed on the second surface 31 and the plurality of second negative signal strips 310 may be formed on the first surface 33.

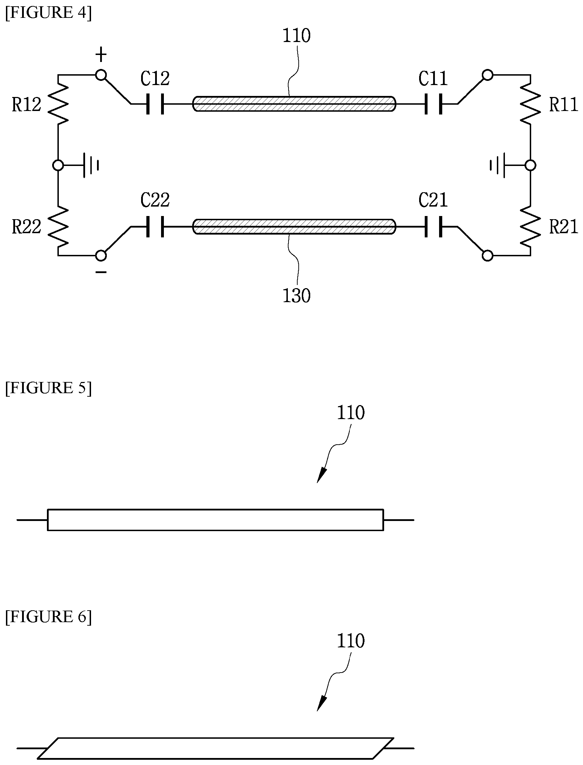

FIG. 3 is an exemplary cross-sectional circuit diagram of the resistance plate detector of FIG. 2, and FIG. 4 is an exemplary plan circuit diagram of the resistance plate detector of FIG. 2.

Although FIGS. 3 and 4 show the first substrate 10, the second substrate 30 may have the same configuration, and the same may apply to the following description.

Referring to FIGS. 3 and 4, each of the first positive signal strip 110 and the first negative signal strip 130 may have two ends connected to at least one resistor and at least one capacitor.

In other words, a capacitor C11 and a resistor R11 are connected to one end of the first positive signal strip 110, and a capacitor C12 and a resistor R12 may be connected to the other end. In addition, a capacitor C21 and a resistor R21 may be connected to one end of the first negative signal strip 130, and a capacitor C22 and a resistor R22 may be connected to the other end.

Although FIGS. 3 and 4 show the first positive signal strip 110 and the first negative signal strip 130, a resistor and a capacitor may be connected to the second positive signal strip 330 and the second negative signal strip 310. In addition, two or more resistors or capacitors may be connected, and a change in location may be made.

FIGS. 5 to 7 show the shapes of the strips of FIG. 2.

In the present disclosure, the first positive signal strip 110 may have a shape of a rectangular that extends in a direction or its variation.

FIG. 5 shows the first positive signal strip 110 having a rectangular shape, and FIG. 6 shows the first positive signal strip 110 having a shape that slantly narrows at one end.

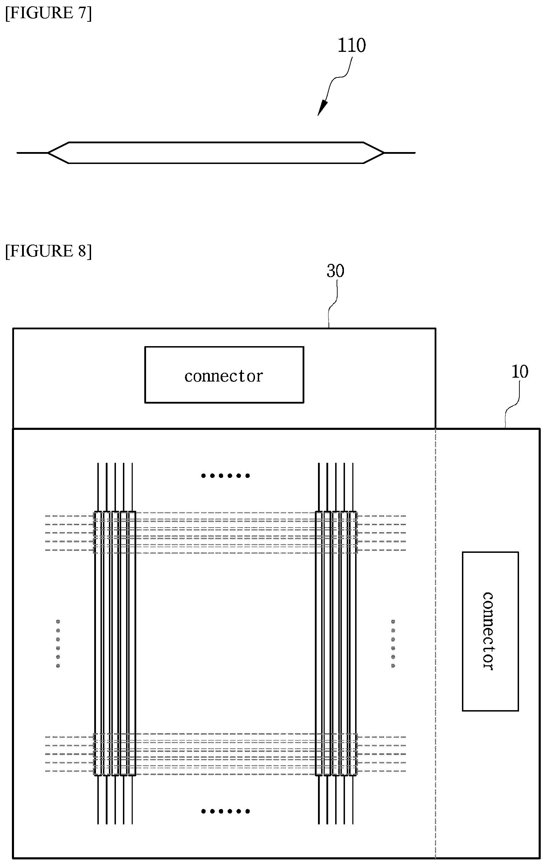

FIG. 7 shows a shape that narrows into a triangular shape at two ends, and in this case, it may be possible to reduce refraction caused by a change in width when a signal is transmitted in a trace.

Although FIGS. 5 to 7 show the first positive signal strip 110, the same may apply to the first negative signal strip 130, the first ground strip 120, the second positive signal strip 330, the second negative signal strip 310 and the second ground strip 320.

FIG. 8 is a schematic plan view of the resistance plate detector of FIG. 2.

Referring to FIG. 8, each of the first substrate 10 and the second substrate 30 may further include a connector. The connector may be formed in an extension portion of the first substrate 10 and the second substrate 30, and the location shown in FIG. 8 is provided for illustrative purposes, and the design may be freely modified to change the location.

The capacitors C11, C12, C21, C22 and the resistors R11, R12, R21, R22 formed at the two ends of the signal strips are connected to the differential amplifier (not shown). The differential amplifier, the capacitors C11, C12, C21, C22 and the resistors R11, R12, R21, R22 may be formed on the connectors formed in the first substrate 10 and the second substrate 30.

The width and interval of the signal strips may be adjusted according to the target input impedance of the differential amplifier.

According to the resistance plate detector for reading each of positive and negative signals with the differential amplifier, as the ground strips are separately formed like the signal strips, interference between channels may be reduced. Accordingly, it may be possible to reduce noise in the resistance plate detector, thereby improving the performance of TOF-PET applied.

Although the present disclosure has been hereinabove described with reference to the embodiments, it will be understood by persons having ordinary skill in the corresponding technical field that a variety of modifications and changes may be made to the present disclosure without departing from the scope of the present disclosure set forth in the appended claims.

### INDUSTRIAL APPLICABILITY

Positron emission tomography (PET) used in the limited applications, for example, Parkinson's disease research early in PET development, operates based on crystal scintillators and optical amplifiers. This system is increasingly being used for cancer diagnosis. PET combined with timing measurement functionality, known as TOF-PET, and whole-body PET are being developed, and they are very expensive.

Recently, it is known that the substitution of resistance plate detectors with crystal scintillators and optical amplifiers may achieve higher performance of TOF-PET at a lower cost. Therefore, it is expected that when the resistance plate detector of the present disclosure is applied to TOF-PET, it is possible to manufacture systems with higher performance at a lower cost.

### [List of Reference Numerals]

1: Resistance plate detector
10: First substrate
30: Second substrate
11: First surface of first substrate
13: Second surface of first substrate
31: Second surface of second substrate
33: First surface of second substrate
110: First positive signal strip
130: First negative signal strip
120: First ground strip
310: Second negative signal strip
330: Second positive signal strip
320: Second ground strip

## Claims

1. A resistance plate detector for reading each of positive and negative signals with a differential amplifier, the resistance plate detector comprising:
a plurality of first positive (+) signal strips formed in a shape of a strip that extends in a first direction on a first surface of a first substrate, and arranged parallel to each other;
a plurality of first negative (-) signal strips formed in a same shape as the first positive signal strips on a second surface opposite the first surface of the first substrate, and arranged parallel to each other in the first direction such that each first negative signal strip and each corresponding first positive signal strip face each other in pair; and
a plurality of first ground strips formed in a same shape between the plurality of first positive signal strips and the plurality of first negative signal strips, and arranged parallel to each other in the first direction,
wherein output signals of the pair of the first positive signal strip and the corresponding first negative signal strip are input to one differential amplifier.

2. The resistance plate detector for reading each of positive and negative signals with the differential amplifier according to claim 1,
wherein each of the plurality of first positive signal strips, the plurality of first negative signal strips and the plurality of first ground strips is formed with a same shape, width, length, thickness and interval.

3. The resistance plate detector for reading each of positive and negative signals with the differential amplifier according to claim 1,
wherein the plurality of first positive signal strips, the plurality of first negative signal strips and the plurality of first ground strips are formed with a same shape, width, length, thickness and interval.

4. The resistance plate detector for reading each of positive and negative signals with the differential amplifier according to claim 1,
wherein each of the first positive signal strip and the first negative signal strip has two ends connected to at least one resistor and at least one capacitor.

5. The resistance plate detector for reading each of positive and negative signals with the differential amplifier according to claim 1,
wherein a width and interval of the plurality of first positive signal strips and the plurality of first negative signal strips is adjusted according to a target input impedance of the differential amplifier.

6. The resistance plate detector for reading each of positive and negative signals with the differential amplifier according to claim 1, further comprising:
a plurality of second positive signal strips formed in a shape of a strip on a first surface of a second substrate and arranged parallel to each other, the first surface of the second substrate spaced a predetermined distance apart from the first surface of the first substrate, facing the first surface of the first substrate;
a plurality of second negative signal strips formed in a same shape as the second positive signal strips on a second surface opposite the first surface of the second substrate, and arranged parallel to each other such that each second negative signal strip and each corresponding second positive signal strip face each other in pair; and
a plurality of second ground strips formed in a same shape between the plurality of second positive signal strips and the plurality of second negative signal strips, and arranged parallel to each other.

7. The resistance plate detector for reading each of positive and negative signals with the differential amplifier according to claim 6,
wherein the plurality of second positive signal strips, the plurality of second negative signal strips and the plurality of second ground strips extend in a second direction perpendicular to the first direction.

8. The resistance plate detector for reading each of positive and negative signals with the differential amplifier according to claim 6,
wherein output signals of the pair of the second positive signal strip and the corresponding second negative signal strip are input to one differential amplifier, and
wherein a width and interval of the plurality of second positive signal strips and the plurality of second negative signal strips is adjusted according to a target input impedance of the differential amplifier.

9. The resistance plate detector for reading each of positive and negative signals with the differential amplifier according to claim 6,
wherein each of the plurality of first positive signal strips, the plurality of first negative signal strips, the plurality of second positive signal strips, the plurality of second negative signal strips, the plurality of first ground strips and the plurality of second ground strips has at least one of a rectangular shape, an extended rectangular shape that narrows into a triangular shape at two ends, or an extended rectangular shape that narrows slantly at one end.

10. The resistance plate detector for reading each of positive and negative signals with the differential amplifier according to claim 1,
wherein the resistance plate detector for reading each of positive and negative signals with the differential amplifier is applied to Time-of-flight (TOF) Positron Emission Tomography (PET).
